(19) Europäisches Patentamt
European Patent Office
Office européen des brevets

(11) Veröffentlichungsnummer : **0 558 956 B1**

(12) # EUROPÄISCHE PATENTSCHRIFT

(45) Veröffentlichungstag der Patentschrift : **20.07.94 Patentblatt 94/29**

(21) Anmeldenummer : **93101919.4**

(22) Anmeldetag : **08.02.93**

(51) Int. Cl.[5] : **C07D 241/44**, C07D 251/18, C07D 251/54, C08K 5/3492, C08L 101/00

(54) **2,3-Dihydroxychinoxalinverbindungen.**

(30) Priorität : **19.02.92 DE 4204970**

(43) Veröffentlichungstag der Anmeldung : **08.09.93 Patentblatt 93/36**

(45) Bekanntmachung des Hinweises auf die Patenterteilung : **20.07.94 Patentblatt 94/29**

(84) Benannte Vertragsstaaten : **AT BE DE FR GB NL**

(56) Entgegenhaltungen : **EP-A- 0 400 600**

(56) Entgegenhaltungen :
**DD-A- 226 244**
**DD-A- 292 621**
**DATABASE WPIL, Week 8143, Derwent Publications Ltd., London, GB**

(73) Patentinhaber : **BASF Aktiengesellschaft**
**Carl-Bosch-Strasse 38**
**D-67063 Ludwigshafen (DE)**

(72) Erfinder : **Aumueller, Alexander, Dr.**
**Rieslingweg 25**
**W-6730 Neustadt (DE)**
Erfinder : **Gareiss, Brigitte, Dr.**
**Schillerstrasse 65**
**W-6700 Ludwigshafen (DE)**

EP 0 558 956 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Additionsverbindungen der allgemeinen Formeln I oder II

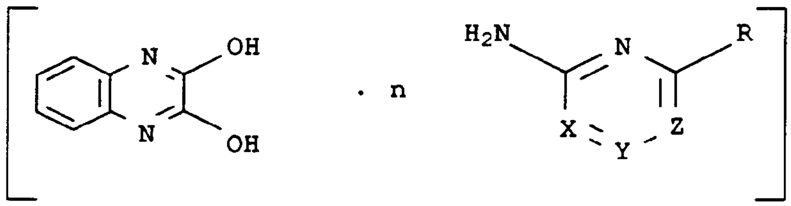

(I)

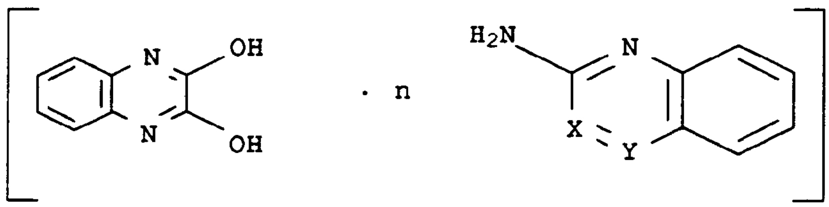

(II)

wobei

n — einen Wert zwischen 0 und 5 hat, und

R — ein Wasserstoffatom, eine $C_1$-$C_8$-Alkylgruppe, eine $C_3$-$C_8$-Cycloalkylgruppe, eine $C_7$-$C_{12}$-Aralkylgruppe, eine $C_6$-$C_{12}$-Arylgruppe, $-NH_2$, -CN oder den Rest eines $C_1$-$C_6$-Alkylesters einer $C_2$-$C_6$-Alkancarbonsäure,

X, Y oder ein Z — Stickstoffatom oder eine Gruppierung der Formel

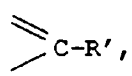

darstellen

R′ — die gleiche Bedeutung wie R haben kann,

wobei zwei benachbarte Gruppen X, Y und Z nicht gleichzeitig ein Stickstoffatom bedeuten.

Des Weiteren betrifft die Erfindung die Herstellung dieser Verbindungen, ihre Verwendung als Flammschutzmittel für thermoplastische Formmassen sowie Formmassen, die die erfindungsgemäßen Additionsverbindungen als Flammschutzmittel enthalten.

Es wurden bereits viele Verfahren vorgeschlagen, um thermoplastische Kunststoffe flammhemmend auszurüsten.

So sind beispielsweise in der EP-A 400 600 Salze des Melamins für diesen Zweck beschrieben. Die US-PS 4 001 177 nennt Derivate des Melamincyanurats als Flammschutzmittel. Diese Flammschutzmittel des Standes der Technik erfüllen die Anforderungen hinsichtlich Eigenfarbe, Thermostabilität, Migration und Ausblüherscheinungen sowie Verträglichkeit mit anderen Zusatzstoffen jedoch nicht in zufriedenstellendem Umfang.

Der Erfindung lag daher die Aufgabe zugrunde, neue Flammschutzmittel für thermoplastische Kunststoffe zur Verfügung zu stellen.

Diese Aufgabe wird erfindungsgemäß durch Additionsverbindungen der allgemeinen Formeln (I) und (II) gelöst,

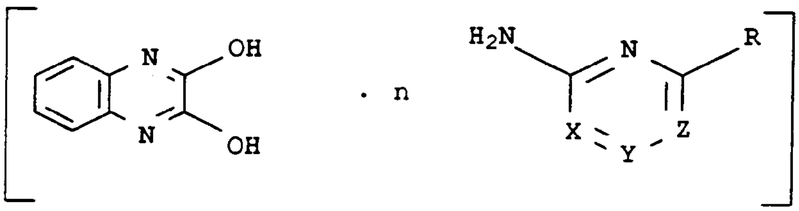

(I)

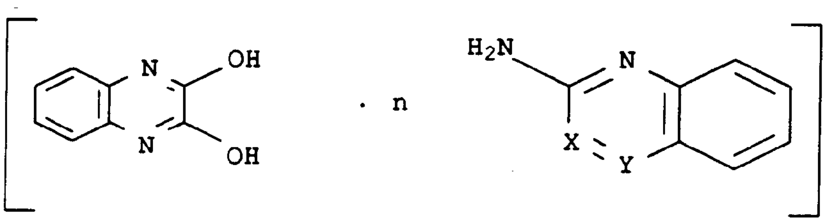

(II)

wobei n einen Wert zwischen 0 und 5, bevorzugt einen Wert im Bereich von 2/3 bis 2 und besonders bevorzugt im Bereich von 2/3 bis 1 darstellt.

Der Substituent R steht für ein Wasserstoffatom, eine $C_1$-$C_8$-, vorzugsweise $C_1$- bis $C_6$-Alkylgruppe, eine $C_3$-$C_8$-, vorzugsweise $C_5$- bis $C_6$-Cycloalkylgruppe, eine $C_6$-$C_{12}$-Aralkylgruppe, eine $C_6$-$C_{12}$-Arylgruppe, oder $-NH_2$, -CN oder den Rest einer $C_1$-$C_6$-Alkylestergruppe einer $C_2$-$C_6$-Alkancarbonsäure.

Diese Verbindungen können allgemein als 2,3-Dihydroxychinoxalinadditionsverbindungen bezeichnet werden.

Bevorzugte Alkylreste sind $C_1$- bis $C_6$-Alkylgruppen wie Methyl, Ethyl, Propyl, iso-Propyl, n-Butyl, iso-Butyl, tert.-Butyl, n-Pentyl, n-Hexyl. Besonders bevorzugt sind Methyl- und Ethylgruppen.

Unter den Cycloalkylgruppen sind bevorzugt Cyclopentyl- und Cyclohexylgruppen zu nennen.

Ein bevorzugter Aralkylrest ist Benzyl und ein bevorzugter Arylrest ist Phenyl.

Auch die Amino und die Cyanogruppe sind bevorzugte Substituenten für R, wobei die Aminogruppe ganz besonders bevorzugt ist.

X, Y, Z bedeuten Stickstoff oder eine

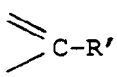 -Gruppe,

wobei R' die gleiche Bedeutung wie die vorstehend genannten Substituenten hat.

Bevorzugt sind X und Z Stickstoff und Y steht für den Rest

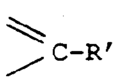 ,

wobei R' eine Amino- oder eine Phenylgruppe ist, besonders bevorzugt eine Aminogruppe.

Ganz besonders bevorzugt als heterocyclische Aminokomponente in den erfindungsgemäßen Additionsverbindungen werden Melamin, Benzoguanamin und 2,4,6-Triaminopyridin.

Die erfindungsgemäßen Additionsverbindungen werden nach dem erfindungsgemäßen Verfahren durch Umsetzung von 2,3-Dihydroxychinoxalin mit Verbindungen der allgemeinen Formel (III) oder (IV)

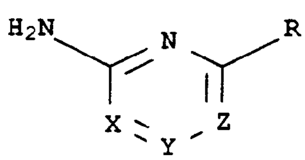

(III)

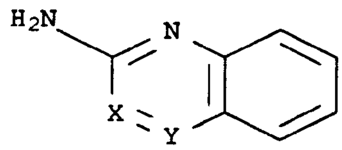

(IV)

vorzugsweise in einem Lösungsmittel, beispielsweise Wasser hergestellt. Die Isolierung der erfindungsgemäßen Additionsverbindungen aus dem Reaktionsgemisch kann durch an sich bekannte Verfahren, beispielsweise Dekantieren, Sprühtrocknen oder Absaugen des Lösungsmittels erfolgen.

Eine weitere Möglichkeit zur Herstellung besteht im intensiven Mischen von 2,3-Dihydroxychinoxalin und Verbindungen der allgemeinen Formeln (III) und (IV) in einer geeigneten Mischvorrichtung, beispielsweise einer Mühle.

Das Molverhältnis der beiden Komponenten liegt zwischen 0 und 5, bevorzugt im Bereich von 2/3 bis 2 und besonders bevorzugt im Bereich von 2/3 bis 1.

Die erfindungsgemäßen Additionsverbindungen eignen sich insbesondere als Flammschutzmittel für thermoplastische Kunststoffe. Zu diesem Zweck können die erfindungsgemäßen Verbindungen den flammhemmend auszurüstenden Formmassen in einer Menge von 1 bis 20 Gew.-%, bevorzugt von 3 bis 15 Gew.-% und besonders bevorzugt von 8 bis 12 Gew.-% zugesetzt werden.

Als thermoplastische Kunststoffe, die durch die erfindungsgemäßen Verbindungen flammhemmend ausgerüstet werden können, seien beispielsweise genannt: Polymere von Mono- und Diolefinen, wie z.B. Polyethylen niedriger oder hoher Dichte, Polypropylen, lineares Polybuten-1, Polyisopren, Polybutadien sowie Copolymerisate von Mono- oder Diolefinen oder Mischungen der genannten Polymere; Copolymerisate von Mono- oder Diolefinen mit Vinylmonomeren, wie z.B. Ethylen-Alkylacrylat-Copolymere, Ethylen-Alkylmethacrylat-Copolymere, Ethylen-Vinylacetat-Copolymere oder Ethylen-Acrylsäure-Copolymere;Polystyrol; Copolymere von Styrol oder α-Methylstyrol mit Dienen oder Acrylderivaten, wie z.B. Styrol-Butadien-, Styrol-Acrylnitril-, Styrol-Ethylmethacrylat-, Styrol-Butadien-Ethylacrylat, Styrol-Acrylnitril-Methacrylat-, ABS-, MBS- oder ähnliche Polymere; halogenhaltige Polymere, wie z.B. Polyvinylchlorid, Polyvinylfluorid, Polyvinylidenfluorid sowie deren Copolymere; Polymere, die sich von α,β-ungesättigten Säuren und deren Derivaten ableiten, wie Polyacrylate, Polymethacrylate, Polyacrylamide und Polyacrylnitrile; Polymere, die sich von ungesättigten Alkoholen und Aminen bzw. von deren Acrylderivaten oder Acetalen ableiten, z.B. Polyvinylalkohol und Polyvinylacetat; Polyurethane, Polyamide, Polyharnstoffe, Polyester, Polycarbonate, Polysulfone, Polyethersulfone und Polyetherketone.

Besonders eignen sich die erfindungsgemäßen Verbindungen als Flammschutzmittel für Polyamide.

Geeignete Polyamide sind an sich bekannt und umfassen die halbkristallinen und amorphen Harze mit Molekulargewichten (Gewichtsmittelwerten) von mindestens 5000, die gewöhnlich als Nylon bezeichnet werden. Solche Polyamide sind z.B. in den amerikanischen Patentschriften 2 071 250, 2 071 251, 2 130 523, 2 130 948, 2 241 322, 2 312 966, 2 516 606 und 3 393 210 beschrieben.

Derartige Polyamide können z.B. durch Kondensation äquimolarer Mengen einer gesättigten oder einer aromatischen Dicarbonsäure mit 4 bis 12 Kohlenstoffatomen mit einem gesättigten oder aromatischen Diamin, welches bis 14 Kohlenstoffatome aufweist oder durch Kondensation von ω-Aminocarbonsäuren oder Polyaddition von Lactamen hergestellt werden.

Beispiele für solche Polyamide sind Polyhexamethylenadipinsäureamid (Nylon 66), Polyhexamethylenazelainsäureamid (Nylon 69), Polyhexamethylensebacinsäureamid (Nylon 610), Polyhexamethylendodecandisäureamid (Nylon 612), die durch Ringöffnung von Lactamen erhaltenen Polyamide wie Polycaprolactam, Polylaurinsäurelactam, ferner Poly-11-aminoundecansäure und Polyamide aus Di(p-aminocyclohexyl)-methan- und Dodecandisäure.

Es ist auch möglich, gemäß der Erfindung Polyamide zu verwenden, die durch Copolykondensation von zwei oder mehr der obengenannten Polymeren oder ihrer Komponenten hergestellt worden sind, z.B. Copolymere aus Adipinsäure, Isophthalsäure oder Terephthalsäure und Hexamethylendiamin oder Copolymere aus Caprolactam, Terephthalsäure und Hexamethylendiamin. Bevorzugt werden lineare Polyamide mit einem Schmelzpunkt über 200°C.

Bevorzugte Polyamide sind Polyhexamethylenadipinsäureamid, Polyhexamethylensebacinsäureamid und Polycaprolactam.

Die Polyamide weisen im allgemeinen Viskositätszahlen im Bereich von 80 bis 250 auf, bestimmt in einer 1 gew.-%igen Lösung in 96 %iger Schwefelsäure bei 23°C, was einem Molekulargewicht von etwa 15 000 bis 45 000 und einer relativen Viskosität, gemessen unter gleichen Bedingungen von 2,0 bis 5,0 entspricht. Polyamide mit einer relativen Viskosität von 2,5 bis 3,5, insbesondere 2,6 bis 3,4 werden bevorzugt verwendet.

Außerdem seien noch Polyamide erwähnt, die z.B. durch Kondensation von 1,4-Diaminobutan mit Adipinsäure unter erhöhter Temperatur erhältich sind (Polyamid-4,6). Herstellungsverfahren für Polyamide dieser Struktur sind z.B. in den EP-A 38 094, EP-A 38 582 und EP-A 39 524 beschrieben.

Die erfindungsgemäßen Additionsverbindungen können den thermoplastischen Kunststoffen auch mit faser- oder teilchenförmigen Füllstoffen zusammen zugemischt werden.

Bevorzugte faserförmige Füllstoffe sind Kohlenstoffasern, Kaliumtitanatwhisker, Aramidfasern und besonders bevorzugt Glasfasern. Bei der Verwendung von Glasfasern können diese zur besseren Verträglichkeit mit den thermoplastischen Kunststoffen mit einer Schlichte und einem Haftvermittler ausgerüstet sein. Im allgemeinen haben die verwendeten Glasfasern einen Durchmesser im Bereich von 6 bis 20 µm.

Die Einarbeitung der Glasfasern kann sowohl in Form von Kurzglasfasern als auch in Form von Endlossträngen (Rovings) erfolgen. Im fertigen Spritzgußteil liegt die mittlere Länge der Glasfasern vorzugsweise im Bereich von 0,08 bis 0,5 mm.

Als teilchenförmige Füllstoffe eignen sich amorphe Kieselsäure, Asbest, Magnesiumcarbonat (Kreide), Kaolin (insbesondere kalzinierter Kaolin), gepulverter Quarz, Glimmer, Talkum, Feldspat und insbesondere Calciumsilikate wie Wollastonit.

Bevorzugte Kombinationen von Füllstoffen sind z.B. 20 Gew.-% Glasfasern mit 15 Gew.-% Wollastonit und 15 Gew.-% Glasfasern mit 15 Gew.-% Wollastonit.

Es versteht sich von selbst, daß auch Mischungen verschiedener Homo- und Copolymerer, gegebenenfalls mit Additiven, Füll- und Verstärkungsstoffen, durch die erfindungsgemäßen Verbindungen flammhemmend ausgerüstet werden können.

Die erfindungsgemäßen Verbindungen können nach allen bekannten Verfahren thermoplastischen Kunststoffen zugesetzt werden. Sie zeichnen sich besonders dadurch aus, daß sie aufgrund ihrer hohen Schmelzpunkte bei hohen Temperaturen eingearbeitet und eingesetzt werden können. So sind sie besonders für Kunststoffe mit hohen Schmelzpunkten geeignet. Ein weiterer Vorteil ist, daß sie mit dem Großteil der bekannten Polymeren kompatibel sind und bei Zugabe die mechanischen Eigenschaften von daraus hergestellten Formteilen nicht oder nur unwesentlich verändern.

Beispiele

1. Herstellung der 2,3-Dihydroxychinoxalinverbindungen

Melamin-2,3-chinoxalindiolat (B1)

648 g 2,3-Dihydroxychinoxalin und 504 g Melamin wurden in 3 l Wasser 5 h auf 100°C erhitzt. Der abgeschiedene Niederschlag wurde nach dem Abkühlen abgesaugt, mit Wasser gewaschen und bei 130°C getrocknet. Man erhielt 1 112 g der Verbindung der Formel

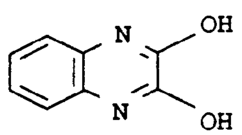

x

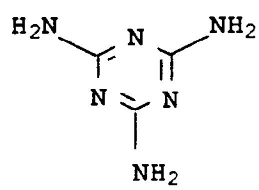

als farblosen Feststoff (Smpkt. 310°C).

Benzoguanamin-2,3-chinoxalindiolat (B2)

486 g 2,3-Dihydroxychinoxalin und 561 Benzoguanamin wurden in 3 l Wasser 5 h auf 100°C erhitzt und wie im vorstehenden Beispiel aufgearbeitet. Man erhielt 1 040 g der Verbindung der Formel

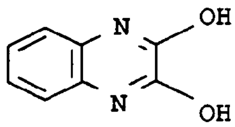 x 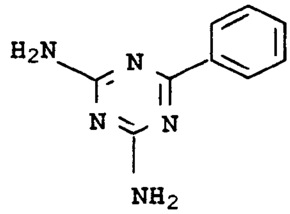

als farblosen Feststoff (Smpkt. 320°C).

2. Verwendung der Verbindungen B1 und B2 in Polyamiden

Eingesetzte Polyamide:

A1: Statistisches Copolyamid, aufgebaut aus 90 Gew.-% Einheiten, die sich von Adipinsäure und Hexamethylendiamin ableiten und 10 Gew.-% Einheiten, die sich von ε-Caprolactam ableiten. Die Viskositätszahl nach ISO 307 betrug 139 ml/g (gemessen in 96 gew.-%iger $H_2SO_4$ als 0,5 gew.-%ige Lösung bei 25°C).

A2: Polyamid-6,6 (Polyhexamethylenadipinsäureamid) mit einer Viskositätszahl nach ISO 307 von 151 ml/g (gemessen in 96 gew.-%iger $H_2SO_4$ als 0,5 gew.-%ige Lösung bei 25°C).

A3: Teilaromatisches Copolyamid, aufgebaut aus 70 Gew.-% Einheiten, die sich von Terephthalsäure und Hexamethylendiamin ableiten und 30 Gew.-% Einheiten, die sich von ε-Caprolactam ableiten. Die Viskositätszahl nach ISO 307 betrug 141 ml/g (gemessen in 96 gew.-%iger $H_2SO_4$ als 0,5 gew.-%ige Lösung bei 25°C).

Komponente B1

Melamin-2,3-chinoxalindiolat

Komponente B2

Benzoguanamin-2,3-chinoxalindiolat

Komponente B3

Melamincyanurat (käuflich)

Die Komponenten wurden auf einem Extruder ZSK 40 (Werner und Pfleiderer, Stuttgart) bei 120 U/min und einem Durchsatz von 30 kg/h konfektioniert. In den Beispielen 1 und 2 wurde bei 260 bis 280°C, in den Beispielen 3 und 4 bei 280 bis 300°C und in den Beispielen 5 und 6 bei 300 bis 330°C konfektioniert und granuliert. Im Vergleichsbeispiel 1 wurde bei 260 bis 280°C, im Vergleichsbeispiel 2 wurde bei 300 bis 330°C konfektioniert. Das Granulat wurde bei 80°C im Vakuum getrocknet und auf einer Spritzgußmaschine zu Probekörpern verarbeitet.

Der Brandtest erfolgte nach UL 94 (Underwriters' Laboratories Inc.) an 1/16 Zoll Prüfkörpern mit üblicher Konditionierung.

Der Elastizitätsmodul wurde nach DIN 53 457, die Schlagzähigkeit nach DIN 53 453 sowie die Schädigungsarbeit (an der Rundscheibe) nach DIN 53 443 gemessen.

Die Zusammensetzung der Formmassen und die Ergebnisse der Messungen sind in der folgenden Tabelle zusammengestellt.

Beispiele:

| Nr. | Zusammensetzung [Gew.-%] | UL 94 | Elastizitätsmodul [N/m²] | Schlagzähigkeit [kJ/m²] bei 23°C | Schädigungsarbeit [Nm] bei 23°C |
|---|---|---|---|---|---|
| 1 | 92 A1, 8 B1 | V-O | 3500 | 25 | 1,20 |
| 2 | 92 A1, 8 B2 | V-O | 3280 | 27 | 0,9 |
| 3 | 92 A2, 8 B1 | V-O | 3600 | 21 | 1,3 |
| 4 | 92 A2, 8 B2 | V-O | 3470 | 18 | 1,0 |
| 5 | 92 A3, 8 B1 | V-O | 3560 | 15 | 0,7 |
| 6 | 92 A3, 8 B2 | V-O | 3430 | 17 | 0,8 |

Vergleichsbeispiele:

| Nr. | Zusammensetzung [Gew.-%] | UL 94 | Elastizitätsmodul [N/m²] | Schlagzähigkeit [kJ/m²] | Schädigungsarbeit [Nm] |
|---|---|---|---|---|---|
| 1 | 92 A1, 8 B3 | V-O | 3540 | - | 0,7 |
| 2 | 92 A3, 8 B3 | nicht konfektionierbar | | | |

**Patentansprüche**

1. Additionsverbindungen der allgemeinen Formeln I oder II

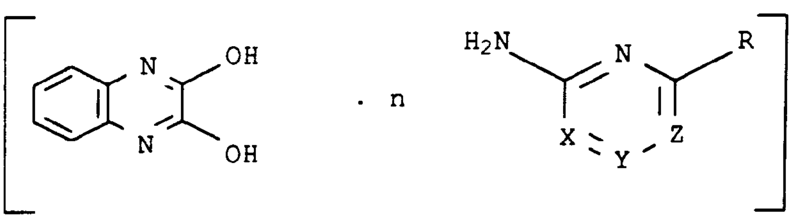

(I)

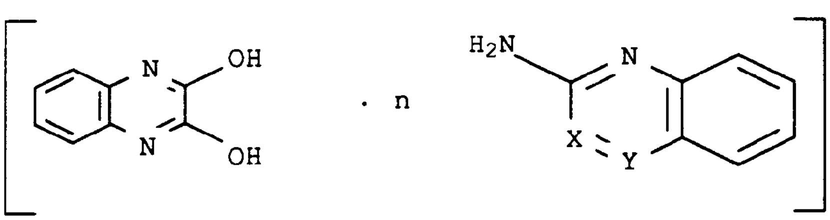

(II)

wobei

| | |
|---|---|
| n | einen Wert zwischen 0 und 5 hat, und |
| R | ein Wasserstoffatom, eine $C_1$-$C_8$-Alkylgruppe, eine $C_3$-$C_8$-Cycloalkylgruppe, eine $C_7$-$C_{12}$-Aralkylgruppe, eine $C_6$-$C_{12}$-Arylgruppe, $-NH_2$, -CN oder den Rest eines $C_1$-$C_6$-Alkylesters einer $C_2$-$C_6$-Alkancarbonsäure, |
| X, Y oder ein Z | Stickstoffatom oder eine Gruppierung der Formel |

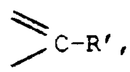

darstellen

| | |
|---|---|
| R′ | die gleiche Bedeutung wie R haben kann, |

wobei zwei benachbarte Gruppen X, Y und Z nicht gleichzeitig ein Stickstoffatom bedeuten.

2. Additionsverbindungen gemäß Anspruch 1, mit der Formel

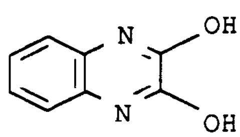

· n

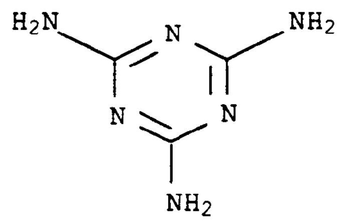

3. Additionsverbindungen gemäß Anspruch 1 mit der Formel

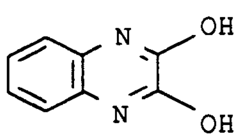

· n

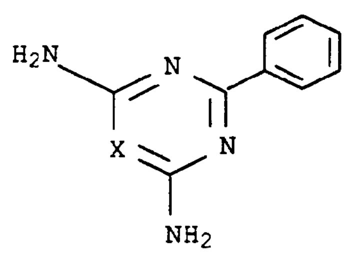

4. Verfahren zur Herstellung von 2,3-Dihydrochinoxalinverbindungen gemäß Anspruch 1, dadurch gekennzeichnet, daß 2,3-Dihydrochinoxalin mit Verbindungen der allgemeinen Formel

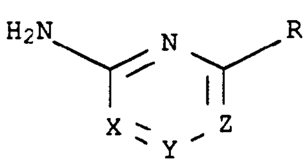

und

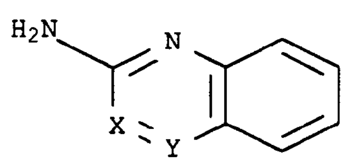

umgesetzt wird.

5. Verwendung von Additionsverbindungen gemäß den Ansprüchen 1 bis 3 als Flammschutzmittel für thermoplastische Formmassen.

6. Thermoplastische Formmassen, enthaltend Additionsverbindungen gemäß nach den Ansprüchen 1 bis 3.

## Claims

1. An adduct of the formula I or II

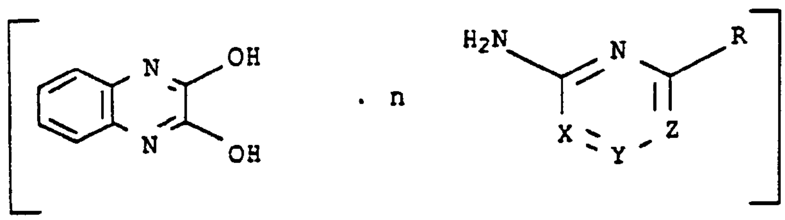

(I)

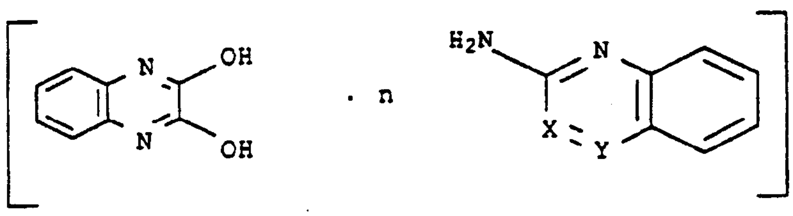

(II)

where
n is from 0 to 5,
R is hydrogen, $C_1$-$C_8$-alkyl, $C_3$-$C_8$-cycloalkyl, $C_7$-$C_{12}$-aralkyl, $C_6$-$C_{12}$-aryl, -$NH_2$, -CN or a radical of a $C_1$-$C_6$-alkyl ester of a $C_2$-$C_6$-alkanecarboxylic acid,
X, Y or Z is a nitrogen atom or a group of the formula

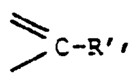

R′ may have the same meanings as R,
two adjacent groups X, Y and Z not simultaneously being a nitrogen atom.

2. An adduct as claimed in claim 1, of the formula

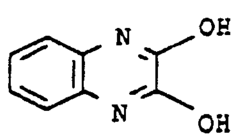

· n

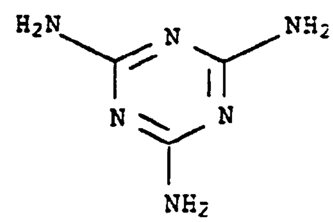

3. An adduct as claimed in claim 1, of the formula

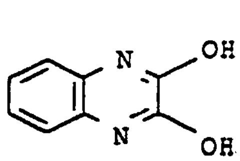

· n

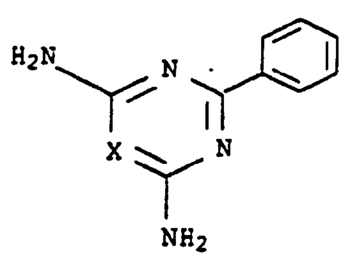

4. A process for the preparation of a 2,3-dihydroquinoxaline compound as claimed in claim 1, wherein 2,3-dihydroquinoxaline is reacted with a compound of the formula

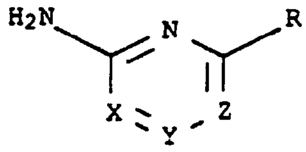
or
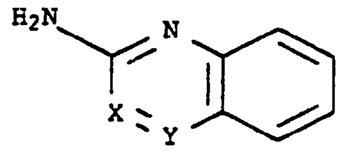

5. Use of an adduct as claimed in any of claims 1 to 3 as a flameproofing agent for thermoplastic molding materials.

6. A thermoplastic molding material containing an adduct as claimed in any of claims 1 to 3.

## Revendications

1. Composés d'addition de formules générales I ou II

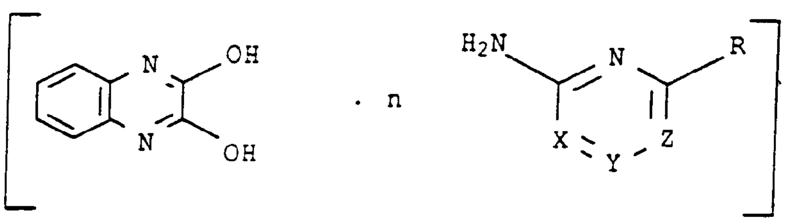
(I)

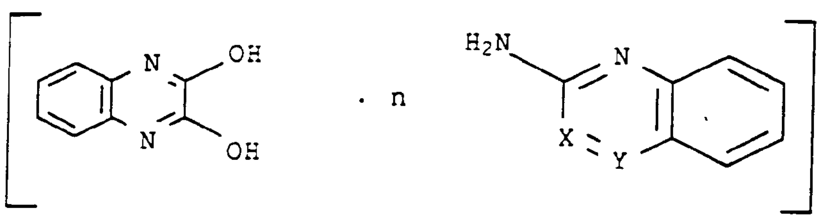
(II)

dans lesquelles

n a une valeur comprise entre 0 et 5, et R représente un atome d'hydrogène, un groupe alkyle en $C_1$-$C_8$, un groupe cycloalkyle en $C_3$-$C_8$, un groupe aralkyle en $C_7$-$C_{12}$, un groupe aryle en $C_6$-$C_{12}$, un groupe $-NH_2$, -CN ou le reste d'un ester d'alkyle en $C_1$-$C_6$ d'un acide alcanecarboxylique en $C_2$-$C_6$,

X, Y ou Z représentent un atome d'azote ou un groupement de formule

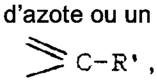
,

R' peut avoir la même signification que R,

deux groupes X, Y et Z voisins ne pouvant pas simultanément représenter un atome d'azote.

2. Composés d'addition selon la revendication 1, ayant la formule

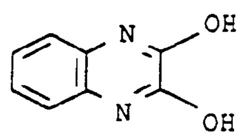

· n

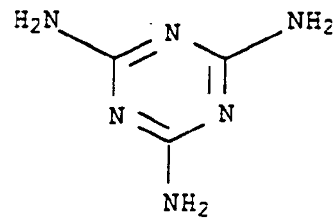

3. Composés d'addition selon la revendication 1, ayant la formule

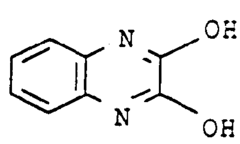

· n

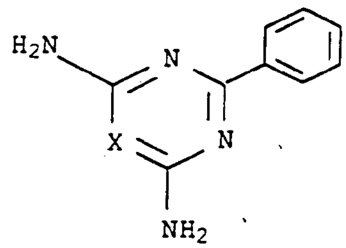

4. Procédé de préparation de composés de 2,3-dihydroxyquinoxaline selon la revendication 1, caractérisé en ce que l'on fait réagir la 2,3-dihydroxyquinoxaline avec des composés de formule générale

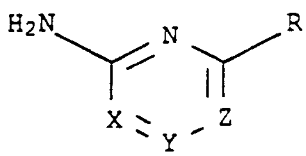

et

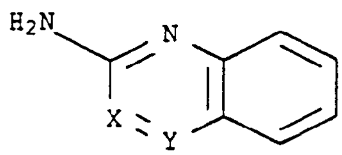

.

5. Utilisation de composés d'addition selon l'une quelconque des revendications 1 a 3 comme agent de protection vis-à-vis des flammes pour des matières à mouler thermoplastiques.

6. Matières à mouler thermoplastiques, contenant des composés d'addition selon l'une quelconque des revendications 1 à 3.